**Europäisches Patentamt**

**European Patent Office**

**Office européen des brevets**

(19)

(11) **EP 0 721 358 B1**

(12) **EUROPEAN PATENT SPECIFICATION**

(45) Date of publication and mention
of the grant of the patent:
**08.05.2002 Bulletin 2002/19**

(51) Int Cl.⁷: **A61M 5/315**

(21) Application number: **94928305.5**

(22) Date of filing: **27.09.1994**

(86) International application number:
**PCT/DK94/00361**

(87) International publication number:
**WO 95/09021 (06.04.1995 Gazette 1995/15)**

(54) **DISPLACEMENT SYSTEM FOR CONTROLLED INFUSION OF A LIQUID**

FÖRDERSYSTEM ZUR GEREGELTEN INFUSION EINER FLÜSSIGKEIT

SYSTEME DE CIRCULATION POUR LA PERFUSION REGULEE D'UN LIQUIDE

(84) Designated Contracting States:
**AT BE CH DE DK ES FR GB GR IE IT LI LU NL PT
SE**

(30) Priority: **27.09.1993 DK 109293**

(43) Date of publication of application:
**17.07.1996 Bulletin 1996/29**

(73) Proprietor: **NOVO NORDISK A/S
2880 Bagsvaerd (DK)**

(72) Inventors:
 • **POULSEN, Jens, Ulrik
  DK-2820 Gentofte (DK)**
 • **LJUNGGREEN, Henrik
  DK-2500 Valby (DK)**
 • **EJLERSEN, Henning, Munk
  DK-2950 Vedbaek (DK)**
 • **MUNK, Jens
  DK-3660 Stenloese (DK)**
 • **KLITMOSE, Lars, Peter
  DK-2820 Gentofte (DK)**
 • **NIELSEN, Preben, Broskov
  DK-2200 Copenhagen N (DK)**
 • **MIKKELSEN, Soren
  DK-2750 Ballerup (DK)**
 • **MOLLER-JENSEN, Jens
  DK-1051 Copenhagen K (DK)**
 • **HEGER, Anders
  DK-2200 Copenhagen N (DK)**

(74) Representative: **Hansen, Einar Tronier et al
Novo Nordisk A/S,
Health Care Patents,
Novo Allé
2880 Bagsvaerd (DK)**

(56) References cited:
**AT-B- 212 625          DE-A- 3 331 424
US-A- 4 493 704**

EP 0 721 358 B1

**Description**

The invention relates to displacement systems for controlled infusion of a liquid from a cartridge of the kind comprising a tubular vessel, which is at a rear end closed by a piston which may be forced by a piston rod moving into the vessel in the axial direction thereof to press out the liquid through an outlet arranged at a front end of the tube, the piston rod being a flexible incompressible construction which at a position behind the rear end of the cartridge is deflected away from the axis of this cartridge by a piston rod guide having a guide track with an upper curved part and being provided behind the rear end of the cartridge and deflecting the piston rod 180° so that parallel portions of the piston rod projects from one side at the piston rod guide, the piston rod being formed by a flexible helix with narrowly adjacent turns of windings, characterised in that the flexible helix has a coiling ratio.

Designated as pumps such displacement systems are used for therapeutic infusion of medicine, e.g insulin for diabetics to whom the natural production of insulin is simulated in. this way.

A commonly used pump structure comprises a housing with a cavity accommodating an infusion syringe cartridge having a piston and a piston rod by which the piston may be forced into the cartridge to press out the medicament in the cartridge through an outlet at the end of the cartridge opposite the piston. Such infusion pumps, which are based on the technique known from syringes by which the dosage can be adjusted with very close accuracy, has many advantages. They are simple in their construction and a precise indication of the medicine left in the cartridge may currently be obtained by monitoring the position of the piston in the cartridge, and in the same way the amount infused can be very precise controlled by controlling the distance of movement of the piston in the cartridge.

A heavy drawback by the pumps of the cartridge and piston type is the fact that at least one of the dimensions of the pump have to be at least more than two times the overall stroke of the piston in the cartridge as the cartridge has to be at least a little longer than this stroke and as space must be reserved for the piston rod behind the cartridge when the piston in a new cartridge is in its rearmost position. Thereby a limit is set for the extent of the miniaturization which is aimed at to make it as comfortable as possible to carry the pump during the activities of a day.

This drawback is avoided by a displacement system of the kind mentioned in the ingress of this specification.

Preferably the piston rod may be a flexible helix with narrowly adjacent turns of windings. This piston rod has no preferred mode of deflection and may be guided in any direction by the piston rod guide.

The deflection of the piston rod away from the axis of the cartridge is obtained by a piston rod guide having a guide track with an upper curved part and being provided behind the rear end of the cartridge. The piston rod guide governs the deflection of the piston rod so that this rod will only be deflected in the way determined by the guide.

Preferably the piston rod is deflected 180° so that it forms two parallel straight portions extending from the piston rod guide.

From DE 33 31 424 A1 and the corresponding SE 449 776 is known a device in which a piston is moved by transmitting a pulling or pressing force through a flexible helix. During the working function of the device the provided flexible piston rod is used for exerting a pulling force on a piston to very slowly suck a fluid, preferably air, into a cylinder ampoule to obtain a sample representative of the ambient air over a time. The piston rod may further be used for pressing the sample out of the cylinder ampoule. It is mentioned that the wire used and the helix must be so dimensioned that the straight portion of the piston rod does not bend out when transmitting a pressing force through this portion and so that the windings are not pulled apart to leave lasting deformation of the helix when a pulling force is transmitted.

Care must be taken to ensure that the helix is so dimensioned that it may transmit an axial pressing force without being compressed and without bending out as any bending out means imprecise dosage. This is avoided by keeping the coiling ratio, i.e. the ratio between the outer diameter of the helix minus the diameter of the wire and the diameter of the wire from which the helix is wound, within certain limits.

Also the initial tension, which keeps the windings of the helix abutting each other even when a pulling force is transmitted by the helix, is of importance to the compressibility of the helix. The larger the initial tension the smaller the tendency for one winding to slip on the adjacent winding to start a bending out. Consequently the initial tension should be maximised.

As the pulling of the piston rod only serves the retraction of the piston rod to its initial position when a new cylinder ampoule is inserted, no heavy pulling force is transmitted and the demands to the precise transmission are low, but the transmission of pressing forces have to be very precise and no compression or bending out of the piston rod can be tolerated.

These problems are all overcome by a displacement system of the kind described in the ingress of this application and which is according to the invention characterised in that the flexible helix has a coiling ratio

$$r_{coil} = \frac{d_{helix} - d_{wire}}{d_{wire}} < 5.0$$

where $d_{helix}$ is the outer diameter of the wound helix, and $d_{wire}$ is the diameter of the wire, and that the guide track has a lover linear part at each end of the upper curved part, and has a length (a) in the direction of the projecting parallel portions, which length taken from the side of the piston rod guide from which these portions projects to the centre of the piston rod at the top of the curved part is equal to or larger than the distance (b) between the axes of the two extending parallel portions.

Preferably $r_{coil}$ is kept < 4,5, more preferably < 4,0, and most preferably < 3,5.

It is also important that the piston rod is abutting the curved guiding surface all the way so that the spring effect of the piston rod does not cause any slack in the guiding as such a slack may result in an imprecise dose. This continuous abutment is obtained by elaborating the curved part of the guide in accordance with the shape which the curved part of the piston rod will spontaneously adopt when its end portions are kept parallel.

When the piston rod is provided as a narrowly wound helix the windings of this helix may present an external thread, which may be engaged by an internally threaded nut element which will drive the piston rod into the cartridge when this nut element is rotated and is not axial displaceable in the housing accommodating the cartridge, the piston rod guide, and a drive mechanism.

The driving force is preferably transmitted to the piston rod at a linear part thereof as the pitch of the rack or the thread are only unambiguously defined at such linear parts.

Preferably the driving force is transmitted to the piston rod immediately behind the cartridge at a position between the cartridge and the piston rod guide. Thereby transmission through the non-linear part of the piston rod is avoided.

Alternatively a driving force may applied by an advancing mechanism at the free end of the piston rod exerting a pressing force in the axial direction of this free end.

Thereby inaccuracies caused by possible compressions in the piston rod are eliminated.

In the following the invention is described in further details with references to the drawing, wherein

Figure 1    schematically shows a known displacement system,

Figure 2    schematically shows a displacement system having a deflectable piston rod.

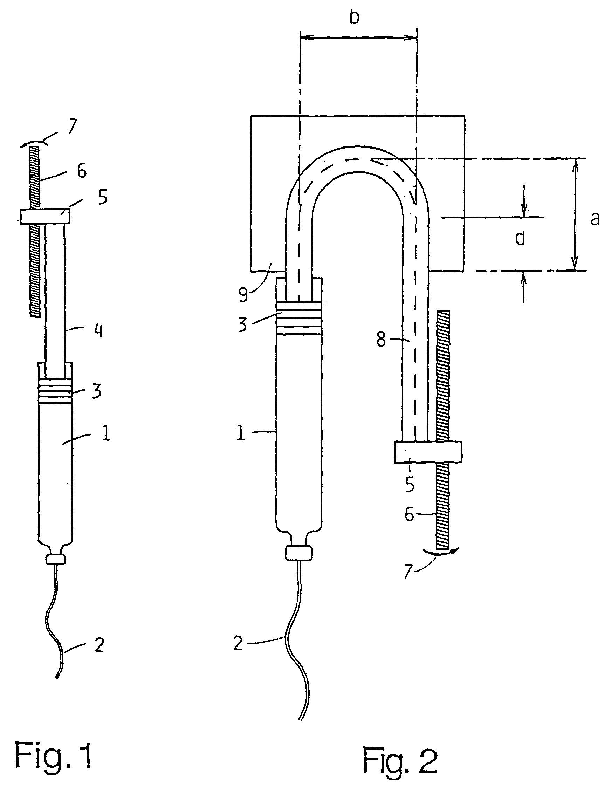

Figure 1 shows schematically a displacement system of the conventional known kind. A cartridge 1 is at one end closed by a closure enabling the mounting of a catheter 2 communicating with a liquid medicine in the cartridge. At its other end the cartridge is closed by a piston 3 which by a piston rod 4 may be pressed into the cartridge to press out the liquid medicine through the catheter 2. The pressure advancing the piston 3 in the cartridge 1 is transmitted to the end of the piston rod through a presser foot 5 which is advanced by a threaded drive spindle by having a bore with an internal thread engaging the thread of the drive spindle 6, which spindle as shown by the arcuate arrow 7 is rotated by a not shown drive mechanism controlling the rate at which the medicine is pressed out through the catheter. The Cartridge 1 and the drive spindle 6 are mounted in a not show housing so that the may not be displaced in relation to each other. The presser foot 5 may abut the end of the piston rod 4 or it may be secured to this end.

A displacement system according to the invention is shown in figure 2. This system comprises to some extent the same elements as do the system shown in figure 1 and these elements are numbered as the corresponding elements in figure 1. However, a flexible piston rod 8 replaces the conventional stiff piston 4 rod of figure 1. Further a piston rod guide 9 is provided which guide deflects the piston rod 8 immediately outside the open end of the cartridge 1.

In the embodiment shown in figure 2 the piston rod 8 is deflected 180 so that the outer end of this flexible rod 8 runs parallel with the cartridge whereby the overall length of the device may be reduced to correspond to about the length of the cartridge and the deflecting piston rod guide 9.

The piston rod guide 9 is equipped with a guiding track conforming the outer contour of the bended or deflected piston rod 8 so that the deflection is guided and no bending of the rod 8 is possible except for the deflection defined by the guide 9.

The guiding track is guiding the piston rod over a length "a" which in the axial direction of the parallel ends of the rod 8 is as long as or longer than the distance "b" between the two axes of the parallel straight portions of the piston rod 8 projecting from the piston rod guide 9. Each end of the guiding track are linear along a distance "d" whereby it is ensured that no deflecting forces have to be exerted on the piston rod 8 outside the piston rod guide 9.

In the embodiment shown in figure 2 the driving force is exerted on the outer end of the deflected piston rod 8. The force is exerted in the axial direction of said outer end and due to the rod 8 being incompressible and the guide guiding the rod conforming with the profile of the flexible rod, the piston rod is displaced along its own axis round through the bending provided by the guide 9 to drive the piston 3 into the cartridge 1. The drive mechanism comprising a presser foot 5 acting on the free end of the piston rod 8 and a threaded spindle 6 engaging an internal thread in a bore in the presser foot 5 may be of any known type providing a rotation in the direction indicated by the arcuate arrow 7.

The flexible piston rod may be advanced by other mechanisms and the driving force may be transmitted to the flexible rod anywhere along its length without deviating from the scope of the invention.

## Claims

1. A displacement system for controlled infusion of a liquid from a cartridge (1) comprising a tubular vessel, which is at a rear end closed by a piston (3) which may be forced by a piston rod (8) moving into the vessel in the axial direction thereof to press out the liquid through an outlet (2) arranged at a front end of the vessel, the piston rod (8) being a flexible incompressible construction which at a position behind the rear end of the cartridge (1) is deflected away from the axis of this cartridge by a piston rod guide (9) having a guide track with an upper curved part and being provided behind the rear end of the cartridge and deflecting the piston rod 180° so that parallel portions of the piston rod projects from one side at the piston rod guide (9), the piston rod being formed by a flexible helix with narrowly adjacent turns of windings, **characterised in that** the flexible helix has a coiling ratio

$$r_{coil} = \frac{d_{helix} - d_{wire}}{d_{wire}} < 5.0$$

where $d_{helix}$ is the outer diameter of the wound helix, and $d_{wire}$ is the diameter of the wire, and that the guide track has a lower linear part at each end of the upper curved part, and has a length (a) in the direction of the projecting parallel portions, which length taken from the side of the piston rod guide from which these portions projects to the centre of the piston rod at the top of the curved part is equal to or larger than the distance (b) between the axes of the two extending parallel portions.

2. A displacement system according to claim 1, **characterised in that** the curved part of the guide track is elaborated in accordance with the shape the curved part of the piston rod (8) will spontaneously adopt when its end portions are kept parallel.

3. A displacement system according to claim 1, **characterised in that** $r_{coil} < 4,5$ more preferably < 4.0 and most preferably < 3,5.

4. A displacement system according to claims 1,2 or 3 **characterised in that** the windings of the helix provides an external thread.

5. A displacement system according to claim 4, **characterised in that** a piston rod drive is provided transmitting a drive force to the piston rod (8) through a nut element having an internal thread engaging the external thread of the piston rod (8).

6. A displacement system according to claim 4 or 5, **characterised in that** the driving force is transmitted to the piston rod (8) at a linear part of this rod (8).

7. A displacement system according any of the claims 1 - 3 **characterised in that** a driving force is exerted on the free, deflected end of the piston rod (8) in the axial direction of this free, deflected end.

## Patentansprüche

1. Fördersystem zur gesteuerten Infusion einer Flüssigkeit aus einer Kartusche (1) mit einem rohrförmigen Gefäß, das an einem hinteren Ende durch einen Kolben-(3) verschlossen ist, der durch eine sich in das Gefäß in axialer Richtung hineinbewegende Kolbenstange (8) druckbeaufschlagt werden kann, um die Flüssigkeit durch einen Auslass (2), der an einem vorderen Ende des Gefäßes angeordnet ist, hinauszupressen, wobei die Kolbenstange (8) eine flexible, nicht komprimierbare Konstruktion ist, welche an einer Position hinter dem rückwärtigen Ende der Kartusche (1) in Richtung von der Achse dieser Kartusche weg umgelenkt ist, und zwar durch eine Kolbenstangenführung (9), deren Führungsbahn einen oberen gekrümmten Teil aufweist und die hinter dem rückwärtigen Ende der Kartusche vorgesehen ist und die Kolbenstange um 180° umlenkt, so dass sich parallele Abschnitte der Kolbenstange aus der einen Seite der Kolbenstangenführung (9) hervorstehen, und die Kolbenstange durch eine flexible Helix mit eng nebeneinander liegenden Wicklungswindungen gebildet ist, **dadurch gekennzeichnet, dass** die flexible Helix folgendes Wicklungsverhältnis aufweist:

$$r_{Wickel} = \frac{d_{Helix} - d_{Draht}}{d_{Draht}} < 5,0$$

wobei $d_{Helix}$ der Außendurchmesser der gewundenen Helix ist, und $d_{Draht}$ der Durchmesser des Drahtes ist, und dadurch, dass die Führungsbahn an jedem Ende des oberen gekrümmten Teils einen unteren linearen Teil aufweist und sie in Richtung der vorstehenden parallelen Abschnitte eine Länge (a) aufweist, welche, gemessen ausgehend von der Seite der Kolbenstangenführung, aus der diese Abschnitte hervorstehen, bis zum Mittelpunkt der Kolbenstange an der Oberseite des gekrümmten Teils, genauso groß oder größer ist als der Abstand (b) zwischen den Achsen der zwei hervorstehenden parallelen Abschnitte.

2. Fördersystem nach Anspruch 1, **dadurch gekennzeichnet, dass** der gekrümmte Teil der Führungsbahn in Übereinstimmung mit der Form ausgearbeitet ist, die der gekrümmte Teil der Kolbenstange (8) spontan annimmt, wenn deren Endabschnitte parallel gehalten werden.

3. Fördersystem nach Anspruch 1, **dadurch gekennzeichnet, dass** $r_{Wickel} < 4,5$ ist, bevorzugter < 4,0 und am bevorzugtesten < 3,5.

4. Fördersystem nach Anspruch 1, 2 oder 3, **dadurch gekennzeichnet, dass** die Wicklungen der Helix ein Außengewinde liefern.

5. Fördersystem nach Anspruch 4, **dadurch gekenn-zeichnet, dass** ein Kolbenstangenantrieb vorgese-hen ist, welcher eine Antriebskraft auf die Kolbenstange (8) über ein Mutterelement überträgt, welches ein Innengewinde aufweist, das mit dem Außengewinde der Kolbenstange (8) in Eingriff ist.

6. Fördersystem nach Anspruch 4 oder 5, **dadurch gekennzeichnet, dass** die Antriebskraft auf die Kolbenstange (8) bei einem linearen Teil dieser Stange (8) übertragen wird.

7. Fördersystem nach einem der Ansprüche 1 bis 3, **dadurch gekennzeichnet, dass** die Antriebskraft auf das freie, umgelenkte Ende der Kolbenstange (8) in Axialrichtung dieses freien, umgelenkten Endes ausgeübt wird.


**Revendications**

1. Système volumétrique de perfusion régulée d'un liquide provenant d'une cartouche (1) comprenant un réservoir tubulaire, qui est fermé à une extrémité arrière par un piston (3) pouvant être forcé par une tige de piston (8) se déplaçant dans le réservoir selon sa direction axiale afin de faire sortir le liquide par pression à travers une sortie (2) disposée à une extrémité avant du réservoir, la tige de piston (8) présentant une construction souple incompressible qui, à une position située derrière l'extrémité arrière de la cartouche (1), est cintrée à l'écart de l'axe de cette cartouche par un guide de tige de piston (9) comportant une voie de guidage dotée d'une partie supérieure courbe, situé derrière l'extrémité arrière de la cartouche et cintrant la tige de piston à 180° de sorte que les parties parallèles de la tige de piston font saillie d'un côté au niveau du guide de tige de piston (9), la tige de piston étant constituée d'une hélice souple dotée de spires d'enroulement étroitement adjacentes, **caractérisé en ce que** l'hélice souple a un rapport de bobinage

$$r_{bobinage} = \frac{d_{hélice} - d_{fil}}{d_{fil}} < 5,0$$

où $d_{hélice}$ est le diamètre extérieur de l'hélice bobinée, et $d_{fil}$ est le diamètre du fil, et **en ce que** la voie de guidage comporte une partie linéaire inférieure à chaque extrémité de la partie courbe supérieure et présente une longueur (a) dans la direction des parties saillantes parallèles, laquelle longueur, prise depuis le côté du guide de tige de piston duquel ces parties font saillie jusqu'au centre de la tige de piston au sommet de la partie courbe, est égale ou supérieure à la distance (b) entre les axes des deux parties s'étendant parallèlement.

2. Système volumétrique selon la revendication 1, **caractérisé en ce que** la partie courbe de la voie de guidage est développée conformément à la forme que la partie courbe de la tige de piston (8) adoptera spontanément lorsque ses parties d'extrémité seront maintenues parallèles.

3. Système volumétrique selon la revendication 1, **caractérisé en ce que** $r_{bobinage} < 4,5$, plus préférablement < 4,0 et le plus préférablement < 3,5.

4. Système volumétrique selon les revendications 1, 2 ou 3, **caractérisé en ce que** les enroulements de l'hélice présentent un filetage externe.

5. Système volumétrique selon la revendication 4, **caractérisé en ce qu'**un mécanisme d'entraînement de tige de piston est prévu et transmet une force d'entraînement à la tige de piston (8) par l'intermédiaire d'un élément formant écrou comportant un filetage interne coopérant avec le filetage externe de la tige de piston (8).

6. Système volumétrique selon la revendication 4 ou 5, **caractérisé en ce que** la force d'entraînement est transmise à la tige de piston (8) au niveau d'une partie linéaire de cette tige (8).

7. Système volumétrique selon l'une quelconque des revendications 1 à 3, **caractérisé en ce qu'**une force d'entraînement est exercée sur l'extrémité libre cintrée de la tige de piston (8) selon la direction axiale de cette extrémité libre cintrée.

Fig. 1

Fig. 2